# EUROPEAN PATENT APPLICATION

(11) **EP 4 738 379 A1**
(43) Date of publication of application: **06.05.2026**
(21) Application number: 25181200.4
(22) Date of filing: 05.06.2025
(51) Int. Cl.: G16H 30/20, G16H 10/60, G06F 16/172

(54) **SYSTEM AND METHOD FOR PRIORITIZING CACHING OPERATIONS BASED ON APPLICATION CONTEXT IN MEDICAL IMAGING PICTURE ARCHIVING AND COMMUNICATION SYSTEMS**

(30) Priority: 05.11.2024 US 202418937822
(71) Applicant: FUJIFILM Healthcare Americas Corporation, Lexington, MA 02421 (US)
(72) Inventor: CARRUTHERS III, William Benjamin, Austin, 78727 (US); VINCENT, Brigil, Chapel Hill, 27517 (US)
(74) Representative: Marks & Clerk LLP

(57) **Abstract**

A method, system and computer program product for prioritized prefetching and caching operations based on a viewer application context within a picture archiving and communication system (PACS). The method includes querying a priority cache for a next imaging study with at least one medical image file requiring prefetching, receiving a next study index corresponding to the imaging study with a highest priority value, a caching operation, and the cache state of the imaging study, identifying the at least one medical image file with the highest priority value requiring prefetching, prefetching the at least one medical image file with the highest priority value requiring prefetching, and storing, in an application cache, the at least one prefetched medical image file of the imaging study with the highest priority value, the application cache corresponding to the associated caching operation and the cache state of the imaging study.

## Description

### TECHNICAL FIELD

The present invention relates to the field of medical picture archiving and communication systems (PACS); and more particularly relates to a system and method for prioritizing prefetching and caching operations based on application context within a PACS.

### BACKGROUND OF THE INVENTION

In the field of medical imaging, the use of PACS has revolutionized the way medical professional's store, access, and analyze patient images. For example, PACS allows healthcare professionals to quickly access and conduct a detailed review of a plurality of medical images in digital format, which can lead to faster and more accurate diagnoses and treatment planning. PACS also allows multiple healthcare professionals to simultaneously access medical images for a particular patient, which provides an efficient way to communicate amongst healthcare professionals and allows for a second opinion in a given medical situation.

However, within a PACS, displaying medical images at interactive frame rates is critical for interpretation workstations, such as radiology workstations. To achieve the fastest initial display and smoothest scrolling performance, all images would ideally be cached locally on the workstation rather than relying on on-demand streaming from a server as the user interacts with the system. One solution is to preemptively download studies to which a user has subscribed based on queries relevant to the user and prioritizing them based on the query order. However, at times, a user may select other studies to open or download that may not be cached on the workstation. For such cases, providing priority retrieval to selected studies may be needed.

Accordingly, there exists a need for improved methods and systems within PACS that enable prioritized prefetching and caching operations based on an application context.

### SUMMARY OF THE INVENTION

It is, therefore, an aspect of the present invention to provide a computer-implemented method for prioritized prefetching and caching operations within a picture archiving and communication system (PACS), the PACS including: a viewer application and a plurality of imaging studies. Each imaging study includes one or more series of medical image files, each series of medical image files including a plurality of medical image files. Each medical image file includes a medical image captured by a medical imaging apparatus and associated metadata. The viewer application interacts with the plurality of medical image files.

The computer-implemented method includes: identifying, by a priority engine of the viewer application, a set of imaging studies of the plurality of imaging studies, wherein each of the set of imaging studies includes at least one medical image file requiring prefetching for a caching operation; determining, by the priority engine, a priority value for each imaging study of the set of imaging studies based on a viewer application context associated with a respective imaging study; storing each imaging study in a study queue, a series queue, or an image queue within a memory of the PACS based on the corresponding priority value, wherein each imaging study is stored with a corresponding study index, a series index, and an image index, a caching operation associated with the at least one medical image file of the imaging study requiring prefetching, and a cache state of the plurality of medical image files of the imaging study; querying, by the priority engine, a priority cache for a next imaging study with at least one medical image file requiring prefetching; receiving, by the priority engine, a next study index corresponding to the imaging study with a highest priority value, an associated caching operation, and the cache state of the imaging study, wherein the next study index is the corresponding study index, the series index, and the image index; identifying, by the priority engine, the at least one medical image file of the imaging study with the highest priority value requiring prefetching; prefetching, by the priority engine, the at least one medical image file of the imaging study with the highest priority value; storing, in an application cache within the memory of the PACS, the at least one prefetched medical image file of the imaging study with the highest priority value, wherein the application cache corresponds to the associated caching operation and the cache state of the imaging study with the highest priority value; fetching at least one medical image file from the application cache when the associated caching operation is to be performed on the at least one medical image file; and executing the associated caching operation on the medical image file.

Another aspect of the present invention provides a computer system for prioritized prefetching and caching operations within a PACS, the PACS comprising: a viewer application and a plurality of imaging studies. Each imaging study includes one or more series of medical image files, each series of medical image files including a plurality of medical image files. Each medical image file includes a medical image captured by a medical imaging apparatus and associated metadata. The viewer application interacts with the plurality of medical image files. The computer system includes one or more computer processors; one or more non-transient computer-readable storage media; program instructions stored on the computer-readable storage media for execution by at least one of the one or more computer processors.

The program instructions include program instructions to: identify, by a priority engine of the viewer application, a set of imaging studies of the plurality of imaging studies, wherein each of the set of imaging studies includes at least one medical image file requiring prefetching for a caching operation; determine, by the priority engine, a priority value for each imaging study of the set of imaging studies based on a viewer application context associated with a respective imaging study; store each imaging study in a study queue, a series queue, or an image queue within a memory of the PACS based on the corresponding priority value, wherein each imaging study is stored with a corresponding study index, a series index, and an image index, a caching operation associated with the at least one medical image file of the imaging study requiring prefetching, and a cache state of the plurality of medical image files of the imaging study; query, by the priority engine, a priority cache for a next imaging study with at least one medical image file requiring prefetching; receive, by the priority engine, a next study index corresponding to the imaging study with a highest priority value, an associated caching operation, and the cache state of the imaging study, wherein the next study index is the corresponding study index, the series index, and the image index; identify, by the priority engine, the at least one medical image file of the imaging study with the highest priority value requiring prefetching; prefetch, by the priority engine, the at least one medical image file of the imaging study with the highest priority value; store, in an application cache within the memory of the PACS, the at least one prefetched medical image file of the imaging study with the highest priority value, wherein the application cache corresponds to the associated caching operation and the cache state of the imaging study with the highest priority value; fetch at least one medical image file from the application cache when the associated caching operation is to be performed on the at least one medical image file; and execute the associated caching operation on the medical image file.

Another aspect of the present invention provides a computer program product system for prioritized prefetching and caching operations within a PACS, the PACS including a viewer application and a plurality of imaging studies, each imaging study including one or more series of medical image files, each series of medical image files including a plurality of medical image files. Each medical image file includes a medical image captured by a medical imaging apparatus and associated metadata. The viewer application interacts with the plurality of medical image files. The computer program product includes a non-transient computer readable storage medium having program code embodied therewith.

The program code executable by a processor to: identify, by a priority engine of the viewer application, a set of imaging studies of the plurality of imaging studies, wherein each of the set of imaging studies includes at least one medical image file requiring prefetching for a caching operation; determine, by the priority engine, a priority value for each imaging study of the set of imaging studies based on a viewer application context associated with a respective imaging study; store each imaging study in a study queue, a series queue, or an image queue within a memory of the PACS based on the corresponding priority value, wherein each imaging study is stored with corresponding study, series, and image indices, a caching operation associated with the at least one medical image file of the imaging study requiring prefetching, and a cache state of the plurality of medical image files of the imaging study; query, by the priority engine, a priority cache for a next imaging study with at least one medical image file requiring prefetching; receive, by the priority engine, a next study index corresponding to the imaging study with a highest priority value, an associated caching operation, and the cache state of the imaging study, wherein the next study index is the corresponding study index, the series index, and the image index; identify, by the priority engine, the at least one medical image file of the imaging study with the highest priority value requiring prefetching; prefetch, by the priority engine, the at least one medical image file of the imaging study with the highest priority value; store, in an application cache within the memory of the PACS, the at least one prefetched medical image file of the imaging study with the highest priority value, wherein the application cache corresponds to the associated caching operation and the cache state of the imaging study with the highest priority value; fetch at least one medical image file from the application cache when the associated caching operation is to be performed on the at least one medical image file; and execute the associated caching operation on the medical image file.

Additional aspects, advantages and novel features of the present invention will be set forth in part in the description which follows and will in part become apparent to those in the practice of the invention, when considered with the attached figures.

### BRIEF DESCRIPTION OF THE DRAWINGS

The present invention will now be described, by way of example, with reference to the accompanying drawings, in which:
FIG. 1 is a block diagram illustrating an information processing environment for anonymous and secure sharing of medical images through a messaging tool within a PACS in accordance with certain embodiments of the present invention;
FIG. 2 is an exemplary screen shot showing a worklist GUI and a viewer GUI in accordance with certain embodiments of the present invention;
FIGS. 3A-3C are flow charts depicting operational steps for prioritizing prefetching and caching operations based on application context within a PACS, in accordance with certain embodiments;
FIGS. 4A-4E are block diagrams illustrating components of the priority engine and the relationship between the priority engine and other components of a PACS, in accordance with some embodiments;
FIG. 5 illustrates caching operation based image, series, and study queue buckets, in accordance with some embodiments; and
FIG. 6 depicts a block diagram of an exemplary computing device that may be used to execute the processes and methods disclosed herein.

Corresponding reference characters indicate corresponding parts throughout the several views. The exemplifications set out herein illustrate currently preferred embodiments of the invention, and such exemplifications are not to be construed as limiting the scope of the invention in any manner.

### DETAILED DESCRIPTION OF THE INVENTION

As will be appreciated by one skilled in the art, aspects of the present invention may be embodied as a system, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer-readable medium(s) having computer-readable program code/instructions embodied thereon.

Any combination of computer-readable media may be utilized. Computer-readable media may be a computer-readable signal medium or a computer-readable storage medium. A computer-readable storage medium may be, for example, but not limited to, an electronic, magnetic, optical, electromagnetic, infrared, or semiconductor system, apparatus, or device, or any suitable combination of the foregoing. More specific examples (a non-exhaustive list) of a computer-readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer-readable storage medium may be any tangible medium that can contain, or store a program for use by or in connection with an instruction execution system, apparatus, or device.

Program code embodied on a computer-readable medium may be transmitted using any appropriate medium, including but not limited to wireless, wireline, optical fiber cable, radio frequency (RF), etc., or any suitable combination of the foregoing.

Computer program code for carrying out operations for aspects of the present invention may be written in any combination of one or more programming languages, including an object-oriented programming language such as Java, Smalltalk, C++ or the like and conventional procedural programming languages, such as the "C" programming language or similar programming languages. The program code may execute entirely on a user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable medium that can direct a computer, other programmable data processing apparatus, or other devices to function in a particular manner, such that the instructions stored in the computer-readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer-implemented process such that the instructions which execute on the computer or other programmable apparatus provide processes for implementing the functions/acts specified in the flowchart and/or block diagram block or blocks.

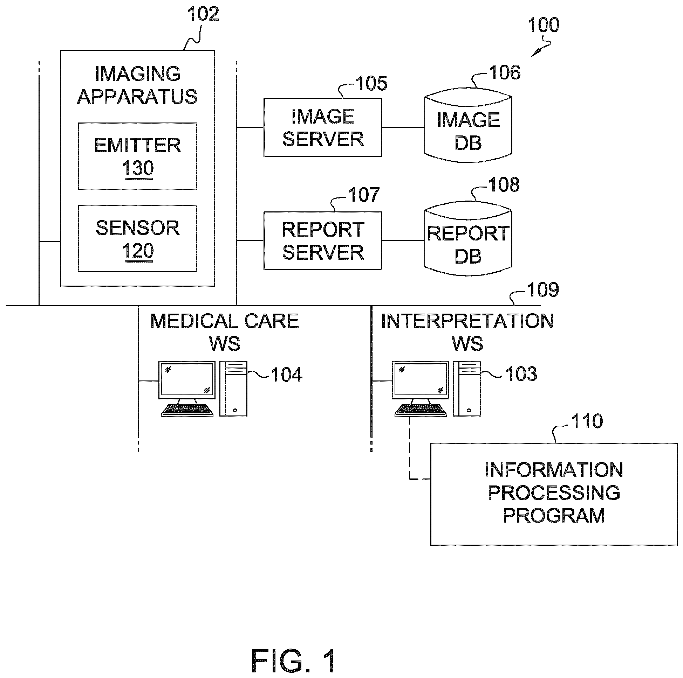

With initial reference to FIG. 1, a block diagram illustrating an information processing environment, generally 100, for capturing, storing, and/or analyzing medical images that can be used in association with an embodiment of the present invention is provided. The information processing environment can include one or more of an imaging apparatus 102, one or more servers (e.g., an image server 105 and a report server 107), and computing devices (e.g., a medical care workstation (WS) 104 and an interpretation WS 103) communicatively coupled together via a network 109. It should be understood that two or more of the aforementioned components may be combined in a single unit. Network 109 can be made up of telecommunication network technologies that are based on physically wired, optical, and/or wireless radio-frequency methods that may be arranged in a variety of network topologies. In general, network 109 can be any set of digital interconnections that allow computing devices to use common communication protocols to communicate with each other.

Imaging apparatus 102 may be one or more imaging devices that include an emitter 130 and a sensor 120. Imaging apparatus 102 is configured to scan the body of a subject by exposing the subject to signals emitted by emitter 130, capturing those signals (or signal reflections) via sensor 120, and thereby obtain detailed internal images of the subject's body (e.g., images of the anatomy and the physiological processes inside the body). The medical images generated by imaging apparatus 102 may be stored in imaging database 106 and/or other database that is communicatively coupled to network 109. For example, imaging apparatus 102 is configured to generate sequential tomographic images of a subject. Imaging apparatus 102 may capture tomographic images along one or more anatomical planes (e.g., the transaxial plane, coronal plane, sagittal plane, median plane, parasagittal plane, and other anatomical planes) of subjects.

Applicable signals emitted by emitter 130 include, but are not limited to, strong magnetic fields, magnetic field gradients, radio waves, X-rays, and ionizing radiation. Imaging apparatus 102 preferably generates heterogeneous sequential tomographic images of the subject. Imaging apparatus 102 can generate medical images using one or more medical imaging techniques that include, but are not limited to, X-ray, CT scan, MRI, PET, MIP, and/or other medical imaging technique capable of generating internal images of a subject's body or part thereof.

The servers may each include a software program providing a database management system (DBMS) installed therein. In some embodiments, image server 105 and report server 107 can be included in one or more computing devices. The servers may include one or more storage media (e.g., flash memory, solid state drive (SSD), or hard disk drive (HDD)) for storing information. For example, image server 105 can include an image database (DB) 106 for at least storing medical images. Report server 107 may include a report DB 108 for at least storing processed reports. Medical care WS 104 can be used by healthcare professionals (e.g., doctors and nurses) to at least observe medical images in detail, view interpretation reports, and/or generate electronic medical records.

Medical care WS 104 may transmit a viewing request for medical images to image server 105, display the received medical images, transmit a viewing request for the associated interpretation report(s) to report server 107, and/or display the received interpretation report. Medical care WS 104 may perform the above processes via executing software programs for respective processes. The steps described in the instant disclosure may be performed by one or more control circuits and/or processors. The interpretation reports can include images analyzed by an information processing program 110 included in interpretation WS 103 (or other computing device communicatively coupled to network 109).

Interpretation WS 103 is a computing device that can be configured to analyze or interpret one or more medical images (e.g., stored in image DB 106 or other data store communicatively coupled to network 109) as described in the present disclosure. Interpretation WS 103 may be used by user (e.g., a healthcare professional such as a radiologist) to interpret one or more medical images and/or create interpretation reports. In certain instances, interpretation WS 103 may be proximate to the other elements in information processing environment 100, such as in a medical center or hospital. In other instances, interpretation WS 103 may be remote to the other elements in information processing environment 100. For example, interpretation WS 103 may be in a home or remote office of a medical professional (e.g., a radiologist). Interpretation WS 103, or other processor(s) communicatively coupled to network 109, can perform steps defined by an information processing program 110. As will be discussed in more detail below, information processing program 110 is computer code that enables the display, navigation, examination, and/or annotation of sets of medical images. Information processing program 110 can be stored in a database communicatively coupled to the interpretation WS 103. Alternatively, information processing program 110 can be stored in one or more databases that are communicatively coupled to network 109.

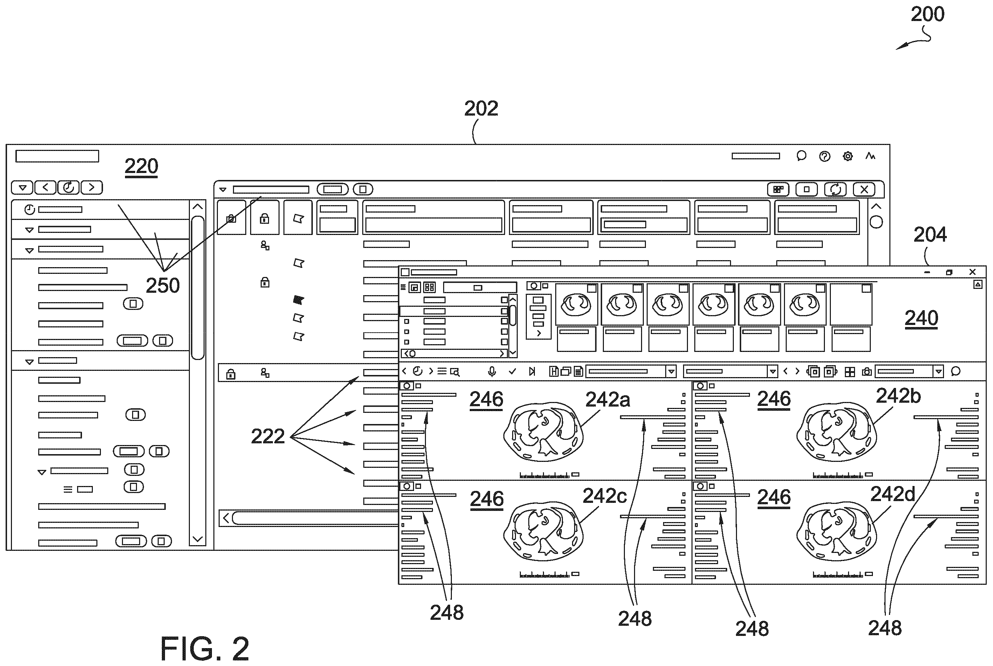

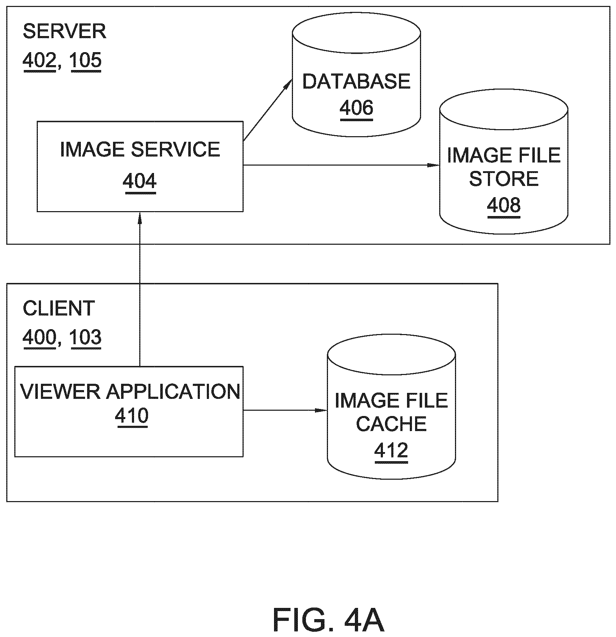

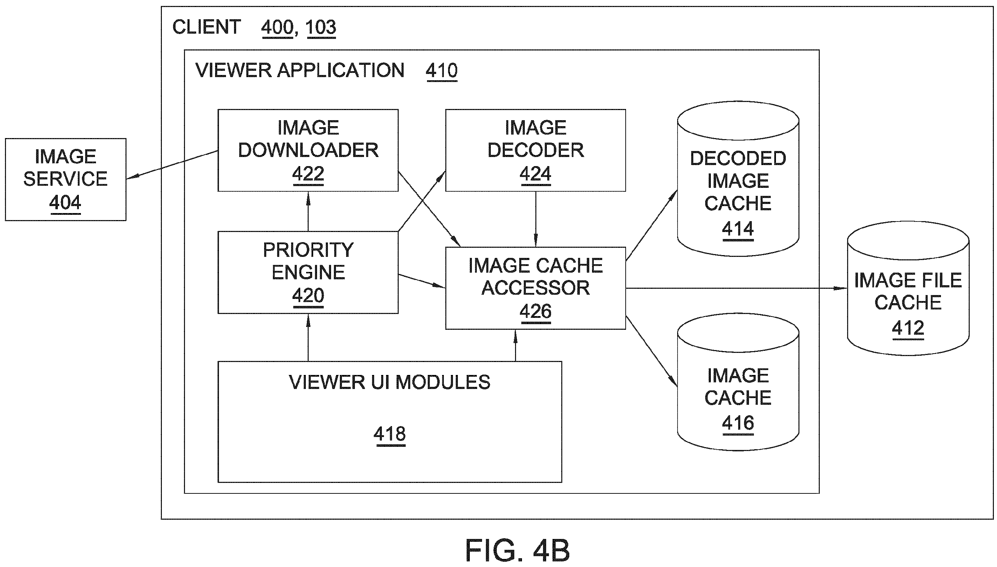

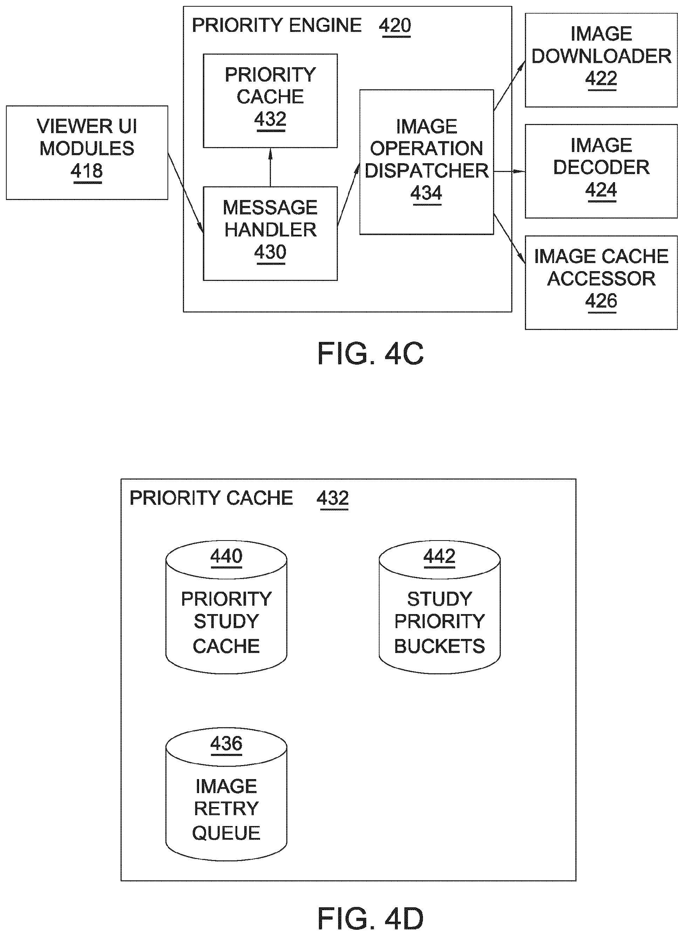

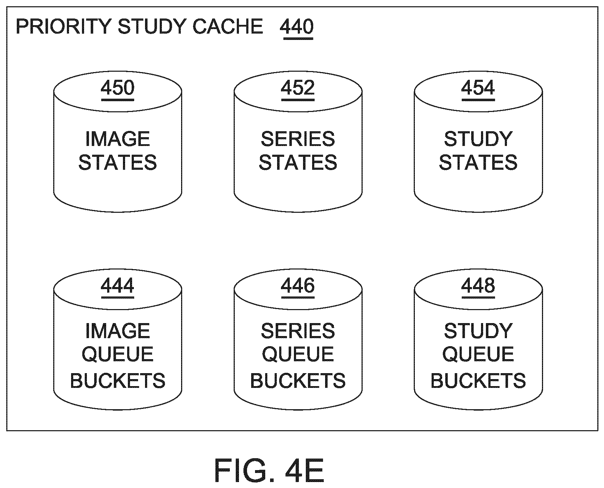

Information processing program 110 may be or may include a picture archiving and communication system (PACS) 200. Referring now to FIGS. 2-5, embodiments of the present invention provide computer-implemented methods, systems, and computer program products for prioritizing prefetching and caching operations based on application context within PACS 200. PACS 200 includes a plurality of imaging studies including a plurality of medical image files, each medical image file including a medical image 242 captured by one or more medical imaging apparatus 102 and stored in one or more databases 106, 108 that are communicatively coupled to network 109. Each of the plurality of medical image files includes a medical image 242 and corresponding metadata 248 (e.g., digital imaging and communications in medicine (DICOM) formatted data). The metadata may comprise a plurality of data objects corresponding to information entities (IEs). For example, the data objects may correspond to information entities such as a patient, a study, a medical device, a physician's visit, a series of images, a patent schedule, a document, a diagnostic interpretation, etc. The data objects may include attributes consisting of a tag, a value representation describing the data type and format, and an alphanumeric data value.

After logging in to PACS 200 on interpretation WS 103 or medical care WS 104, a worklist graphical user interface (GUI) 220 may be provided in a first window 202 displayed on a display of WS 103, 104. Worklist GUI 220 may include one or more panes 250 displaying one or more lists of a plurality of imaging studies 222. Each of plurality of imaging studies 222 may be associated with one or more medical image files, each medical image file including a medical image 242 of a subject (e.g., person) captured by one or more medical imaging apparatuses 102.

When an imaging study 222 is selected from worklist GUI 220, a second window 204 providing a viewer GUI 240 may be displayed on the display of WS 103, 104. Viewer GUI 240 includes at least one pane 246 that is configured for displaying at least one medical image 242 of the selected at least one imaging study 222. The at least one medical image 242 is displayed in at least one pane 246 for viewing by medical professionals for the purpose of reviewing, evaluating and diagnosing any medical conditions that may be present in the images subject. The at least one medical image 242 includes a metadata overlay layer comprising metadata 248.

In some embodiments, viewer GUI 240 may include a plurality of panes 246, wherein each pane displays an image of the plurality of medical images 242 of selected imaging study 222.

As noted above, within a PACS, displaying medical images at interactive frame rates is critical for interpretation workstations. Medical image file sizes within a PACS can range from 1 kilobyte to hundreds of megabytes depending on the type of procedure and body part being imaged. To achieve the fastest initial display and smoothest scrolling performance, the images a user selects to view would ideally be cached locally on the workstation (e.g., WS 103,104) rather than relying on on-demand streaming from a server as the user interacts. To accomplish this fast initial display and smooth scrolling performance, subscribed studies may be preemptively download based on queries relevant to the user and prioritizing them based on the query order. However, as PACS 200 is being used, a user may select other studies, that may not be cached on the workstation, to open or download. For such cases, priority to selected studies may be needed.

Ultimately, the system must determine an optimal next image and operation to dispatch, given application context, subscriptions, and resource constraints such as CPU cores and storage space. The system should achieve eventual consistency and minimize the possibility of cyclical caching and flushing operations. The system must also have flexibility to support the DICOM information model, allowing priorities specified at various hierarchy levels.

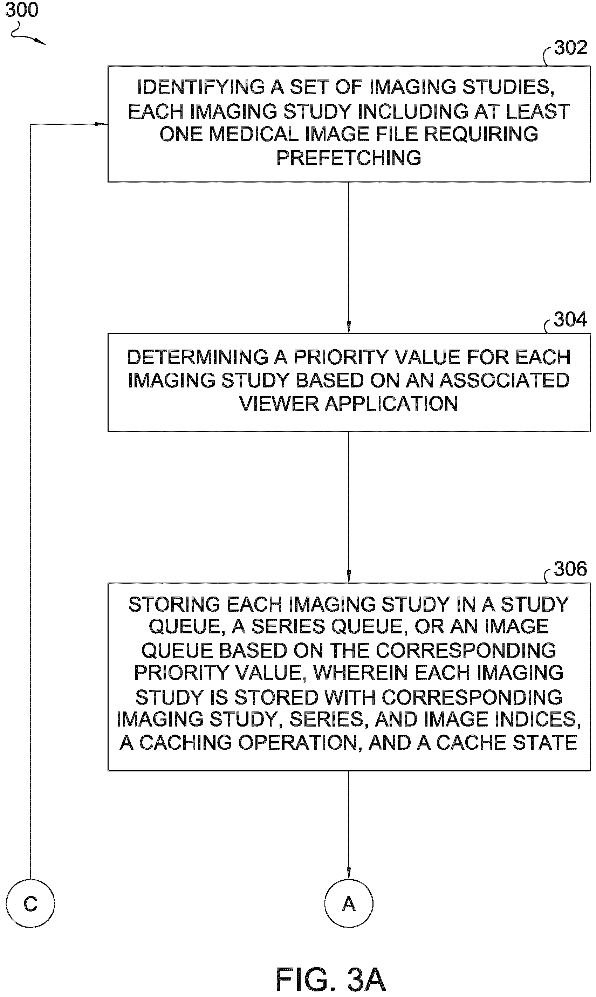

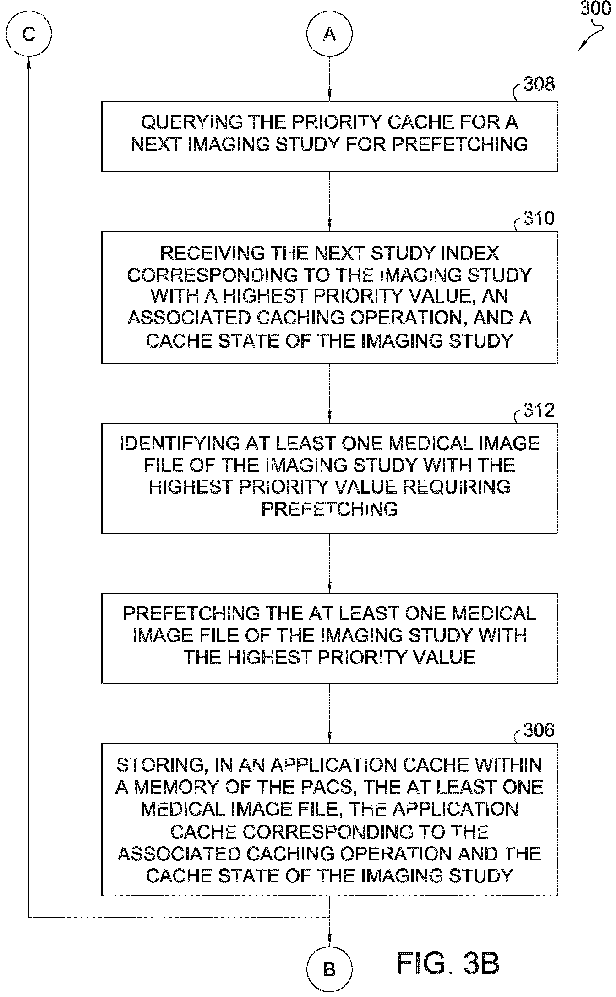

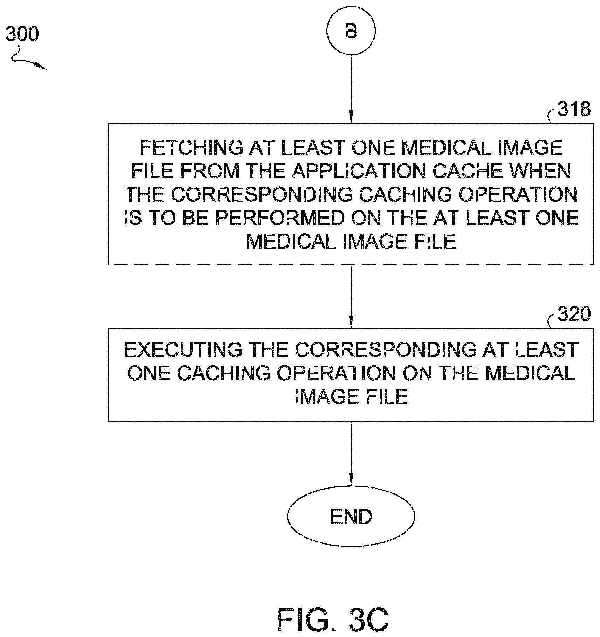

With additional reference to FIGS. 3A-5, embodiments of the present invention provide computer-implemented methods, systems, and computer program products for prioritized prefetching and caching operations based on a viewer application context within PACS 200. PACS 200 includes a viewer application 410 hosted on a client device 400 (e.g., interpretation WS 103 or medical care WS 104) and a plurality of imaging studies 222 stored in an image file store 408 and managed by a database 406 (e.g., image DB 106) on one or more servers 402 (e.g., image server 105). Image service 404 may be used by one or more electronic devices (e.g., imaging apparatus 102, medical care WS 104 and interpretation WS 103, etc.) to access image metadata from database 406 and to read image medical image files from or write medical image files to an image file store 408. Each imaging study 222 may include one or more series of medical image files. Each series of medical image files may include a plurality of medical image files. Each medical image file includes a medical image 242 captured by a medical imaging apparatus (e.g., imaging apparatus 102) and associated metadata (e.g., DICOM formatted data). Viewer application 410 interacts with the plurality of medical image files, prefetching and caching the medical image files in image file cache 412.

With additional reference to a computer-implemented method 300 shown in FIGS. 3A-3C, viewer application 410 includes a priority engine 420 that identifies, at operational step 302, a set of imaging studies of the plurality of imaging studies 222, wherein each of the set of imaging studies includes at least one medical image file requiring prefetching for a caching operation. The aforementioned identification may include identifying a first set of imaging studies indicated by one or more user queries, identifying a second set of imaging studies indicated by one or more user inputs selecting at least one imaging study, and identifying a third set of imaging studies indicated by a user subscription, and aggregating the first set of imaging studies, the second set of imaging studies, and the third set of imaging studies into the set of imaging studies of the plurality of imaging studies. It should be understood that a user may query imaging studies, select imaging studies or medical images within an imaging study, and subscribe to imaging studies via viewer user interface (UI) modules 418 (e.g., worklist GUI 220 and viewer GUI 240).

At operational step 304, priority engine 420 determines a priority value for each imaging study of the set of imaging studies based on a viewer application context associated with a respective imaging study. The priority value for each imaging study may include at least one of an image level priority value, a series level priority value, or a study level priority value, wherein the image level priority value is higher in priority than the series level priority value and the series level priority value is higher in priority than the study level priority value. Additionally, the viewer application context associated with the respective imaging study includes an associated caching operation to be performed on at least one medical image of the imaging study, a cache state of the plurality of medical image files of the imaging study, and data indicating whether there is a user input or a user query associated with the imaging study.

At operational step 306, each imaging study in the set of imaging studies is stored by priority engine 420 in a study queue bucket 448, a series queue bucket 446, or an image queue bucket 444 of a priority study cache 440 within priority cache 432 based on the corresponding priority value. Each imaging study may be stored with corresponding study, series, and image indices, a caching operation associated with the at least one medical image file of the imaging study requiring prefetching, and a cache state of the plurality of medical image files of the imaging study. The cache state may be at least one of an image cache state 450, a series cache state 452, or a study cache state 454. The cache state may include data indicating whether there are active operations for medical image files associated with the image study, data indicating whether the image study and the associated medical image files are present in the application cache, data indicating eligibility of the image study and the associated medical image files for each caching operation.

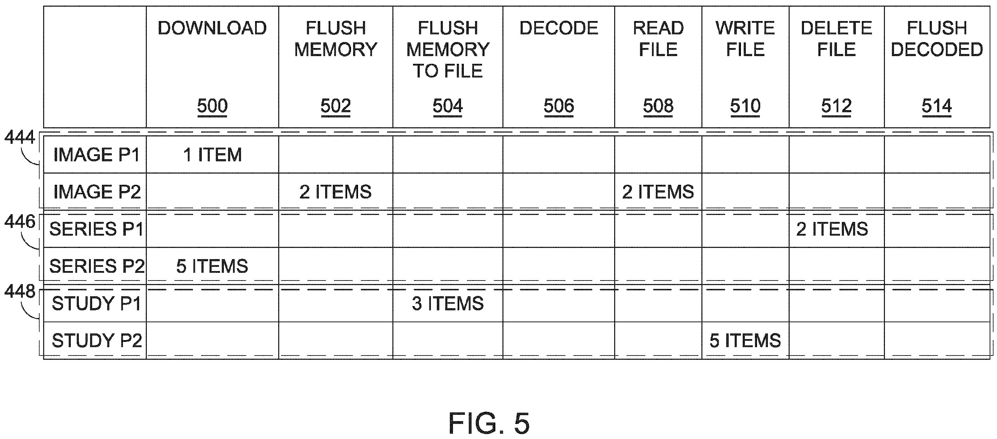

With additional reference to caching operation based image, series, and study queue buckets illustrated in FIG. 5, caching operations may include downloading an object 500 (e.g., a medical image file), flushing an image file from a specific memory cache 502, flushing an image file from a specific memory cache to a file 504, decoding a medical image file 506, reading a stored medical image file 508, writing a medical image file to memory 510, deleting a medical image file 512, flushing a decoded medical image file 514, and the like. Each caching operation may have an associated operation cache bucket associated with each of the various priority levels. For example, each caching operation may have an operation cache bucket associated with each image level priority value (e.g., image level priority value P1, image level priority value P2, et cetera), a cache bucket associated with each series level priority value (e.g., series level priority value P1, series level priority value P2, etc.), and a cache bucket associated with each study level priority value (e.g., study level priority value P1, study level priority value P2, et cetera). In certain embodiments, a round-robin ordering of operations may be utilized for items (i.e., image of an imaging study needing to be prefetched) with equal priority levels.

Within a caching operation, image level priority values are higher in priority than series level priority values, and series level priority value are higher in priority than study level priority values. In some instances, a caching operation may have a priority bucket with more than one item having a same priority level. For example, flushing memory operation 502 may have two items with an image level priority value of P2 or download operation 500 may have five items with a series level priority value of P2.

At operational step 308, priority engine 420 queries priority cache 432 for a next imaging study with at least one medical image file requiring prefetching. To allow efficient queries of the next object/operation pair to dispatch, the system maintains caches of object/operation eligibility at various hierarchy levels within priority cache 432. Priority cache 432 may be a data structure including priority study cache 440, study priority buckets 442, an image retry queue 436, and the like. Study priority buckets 442 may include a queue of imaging study indices corresponding to medical studies including at least one medical image needing to be prefetched. A study priority bucket may correspond to each caching operation for each priority level. For example, each cell of FIG. 5 may correspond to a study priority bucket. Image retry queue 436 may include, for example, items that were unsuccessfully prefetched.

At operational step 310, priority engine 420 receives a next study index or identifier corresponding to the imaging study with a highest priority value, an associated caching operation, and the cache state of the imaging study. A study index or identifier may include a corresponding study index or identifier, a series index or identifier, and an image index or identifier corresponding to the imaging study. The indices may include one or more alpha-numeric characters. In some embodiments, subsequent image indices within a series may be sequential or alphabetical, and subsequent series indices within a study may be sequential or alphabetical.

At operational step 312, priority engine 420 identifies the at least one medical image file of the imaging study with the highest priority value requiring prefetching. If the highest priority value is an image level priority value, then the indicated image is immediately returned. If the highest priority value is a series level priority value, the series queue within the study is queried for the next series, the range of image indices the series covers within the study is retrieved, within the range of image indices the images buckets of the study are queried, and the first image of the series within the study needing to be prefetched is returned. If the highest priority value is a study level priority value, the images buckets of the study are queried and the first image of the series within the study needing to be prefetched is returned.

At operational step 314, priority engine 420 prefetches the at least one medical image file of the imaging study with the highest priority value. A message handler 430 of priority engine 420 sends an operation and an image to an image operation dispatcher 434. Image operation dispatcher 434 sends the operation and the image to an image downloader 422, an image decoder 424, an image cache accessor 426, or the like based on the operation.

At operational step 316, the at least one prefetched medical image file of the imaging study with the highest priority value is returned to image cache accessor 426 and stored in an application cache (e.g., image file cache 412, a decoded image cache 414, an image cache 416, or the like) within a memory of the PACS (e.g., a system memory 636). The application cache corresponds to the associated caching operation and the cache state of the imaging study with the highest priority value. After operational step 316, computer-implemented method 300 may repeatedly return to operational step 302 to update the priority values in priority cache 420 and query priority cache 420 for the next caching operation to perform and dispatch those operations while proceeding in parallel to operational step 318. After a medical image file is prefetched and stored in an application cache, one or more cache states may be updated to reflect updated states and eligibilities.

At operational step 318, at least one medical image file from the application cache is fetched when the associated caching operation is to be performed on the at least one medical image file. At operational step 320, the associated caching operation is executed on the medical image file.

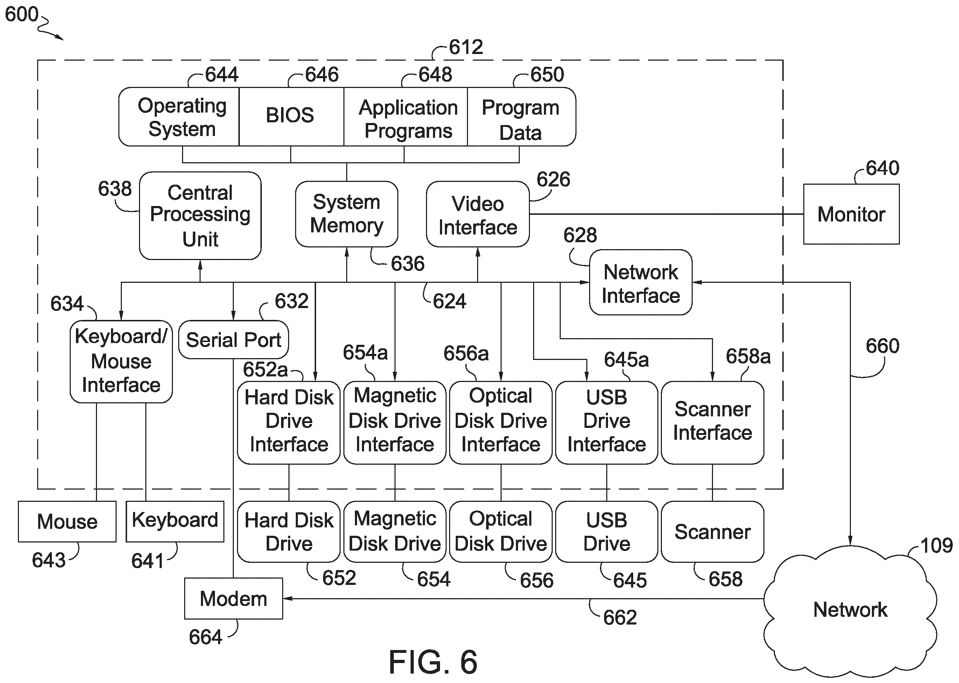

Referring to FIG. 6, an exemplary computing environment 600 is shown that can be utilized through programming to implement any of the processing thus far described. The computing environment 600 may comprise a computer 612 including a system bus 624 that couples a video interface 626, network interface 628, one or more serial ports 632, a keyboard/mouse interface 634, and a system memory 636 to a Central Processing Unit (CPU) 638. Computer 612 may also include a Graphics Processing Unit (GPU) or one or more other special or general purpose processing units. A monitor or display 640 is connected to bus 624 by video interface 626 and provides the user with a graphical user interface to view, edit, and otherwise manipulate items displayed on computer 612. The graphical user interface allows the user to enter commands and information into computer 612 using a keyboard 641 and a user interface selection device 643, such as a mouse or other pointing device. Keyboard 641 and user interface selection device are connected to bus 624 through keyboard/mouse interface 634. The display 640 and user interface selection device 643 are used in combination to form the graphical user interface which may allow a user to implement at least a portion of the present invention. Other peripheral devices may be connected to computer 612 through serial port 632 or universal serial bus (USB) drives 645 to transfer information to and from computer 612.

The system memory 636 is also connected to bus 624 and may include ROM, RAM, an operating system 644, a basic input/output system (BIOS) 646, application programs 648 and program data 950. The computer 612 may further include a hard disk drive 652 for reading from and writing to a hard disk, a magnetic disk drive 654 for reading from and writing to a removable magnetic disk (e.g., floppy disk), and an optical disk drive 956 for reading from and writing to a removable optical disk (e.g., CD ROM or other optical media). The computer 612 may also include USB drives 645 and other types of drives for reading from and writing to flash memory devices (e.g., compact flash, memory stick/PRO and DUO, SD card, multimedia card, smart media card), and a scanner 958 for scanning items such as digital images to be downloaded to computer 612. A hard disk interface 652a, magnetic disk drive interface 654a, an optical drive interface 656a, a USB drive interface 645a, and a scanner interface 658a operate to connect bus 624 to hard disk drive 652, magnetic disk drive 654, optical disk drive 656, USB drive 645 and a scanner 658, respectively. Each of these drive components and their associated computer-readable media may provide computer 612 with non-volatile storage of computer-readable instruction, program modules, data structures, application programs, an operating system, and other data for the computer 612. In addition, it will be understood that computer 612 may also utilize other types of computer-readable media in addition to those types set forth herein, such as digital video disks, random access memory, read only memory, other types of flash memory cards, magnetic cassettes, and the like.

Network interface 628 provides a communication path 660 between bus 624 and network 109, which allows notifications, information and other data to be communicated through network 109 from any of the previously identified devices, and optionally saved in a memory, to the computer 612. This type of logical network connection is commonly used in conjunction with a local area network. Images may also be communicated from bus 624 through a communication path 662 to network 109 using serial port 632 and a modem 664. Using a modem connection between the computer 612 and other computing devices, databases, or the like may be used in conjunction with a wide area network or the Internet. It will be appreciated that the network connections shown herein are merely exemplary, and it is within the scope of the present invention to use other types of network connections between computer 612 and other computing devices including both wired and wireless connections.

As discussed above, embodiments of the present invention provide improved methods and communication systems within PACS that enable prioritized prefetching and caching operations based on application context.

Based on the foregoing, method, computer system, and program product have been disclosed in accordance with the present invention. However, numerous modifications and substitutions can be made without deviating from the scope of the present invention. Therefore, the present invention has been disclosed by way of example and not limitation.

## Claims

1. A computer system for prioritized prefetching and caching operations within a picture archiving and communication system (PACS), the PACS comprising: a viewer application and a plurality of imaging studies, each imaging study including one or more series of medical image files, each series of medical image files including a plurality of medical image files, wherein each medical image file includes a medical image captured by a medical imaging apparatus and associated digital imaging and communications in medicine (DICOM) data, wherein the viewer application interacts with the plurality of medical image files, the computer system comprising:
one or more computer processors;
one or more non-transient computer-readable storage media;
program instructions stored on the computer-readable storage media for execution by at least one of the one or more computer processors, the program instructions comprising:
program instructions to:
identify, by a priority engine of the viewer application, a set of imaging studies of the plurality of imaging studies, wherein each of the set of imaging studies includes at least one medical image file requiring prefetching for a caching operation;
determine, by the priority engine, a priority value for each imaging study of the set of imaging studies based on a viewer application context associated with a respective imaging study;
store each imaging study in a study queue, a series queue, or an image queue within a memory of the PACS based on the corresponding priority value, wherein each imaging study is stored with:
a corresponding study index, a series index, and an image index,
a caching operation associated with the at least one medical image file of the imaging study requiring prefetching, and
a cache state of the plurality of medical image files of the imaging study;
query, by the priority engine, a priority cache for a next imaging study with at least one medical image file requiring prefetching;
receive, by the priority engine, a next study index corresponding to the imaging study with a highest priority value, an associated caching operation, and the cache state of the imaging study, wherein the next study index is the corresponding study index, the series index, and the image index;
identify, by the priority engine, the at least one medical image file of the imaging study with the highest priority value requiring prefetching;
prefetch, by the priority engine, the at least one medical image file of the imaging study with the highest priority value;
store, in an application cache within the memory of the PACS, the at least one prefetched medical image file of the imaging study with the highest priority value, wherein the application cache corresponds to the associated caching operation and the cache state of the imaging study with the highest priority value;
fetch at least one medical image file from the application cache when the associated caching operation is to be performed on the at least one medical image file; and
execute the associated caching operation on the medical image file.

2. The computer system of claim 1, wherein the program instructions to identify the set of imaging studies of the plurality of imaging studies comprise program instructions to:
identify a first set of imaging studies within the set of imaging studies, the first set of imaging studies including at least one medical image file requiring prefetching for a first caching operation indicated by one or more user queries;
identify a second set of imaging studies within the set of imaging studies, the second set of imaging studies including at least one medical image file requiring prefetching for a second caching operation indicated by one or more user inputs selecting at least one imaging study; or
identify a third set of imaging studies within the set of imaging studies, the third set of imaging studies including at least one medical image file requiring prefetching for a third caching operation indicated by a user subscription.

3. The computer system of claim 2, wherein the program instructions to identify the set of imaging studies of the plurality of imaging studies further comprise program instructions to:
aggregate the first set of imaging studies, the second set of imaging studies, and the third set of imaging studies into the set of imaging studies of the plurality of imaging studies.

4. The computer system of claim 1, wherein the priority value for each imaging study includes at least one of an image level priority value, a series level priority value, or a study level priority value, and
wherein the image level priority value is higher than the series level priority value, and wherein the series level priority value is higher than the study level priority value.

5. The computer system of claim 1, wherein the viewer application context associated with the respective imaging study includes the associated caching operation, the cache state of the plurality of medical image files of the imaging study, and data indicating whether there is a user input or a user query associated with the imaging study.

6. The computer system of claim 1, wherein the cache state of the image study comprises:
data indicating whether there are active operations for medical image files associated with the image study;
data indicating whether the image study and the associated medical image files are present in the application cache; or
data indicating eligibility of the image study and the associated medical image files for each caching operation.

7. The computer system of claim 1, wherein the application cache comprises at least one of a decode image cache, an image file cache, or an image cache.

8. A computer program product for prioritized prefetching and caching operations within a picture archiving and communication system (PACS), the PACS comprising: a viewer application and a plurality of imaging studies, each imaging study including one or more series of medical image files, each series of medical image files including a plurality of medical image files, wherein each medical image file includes a medical image captured by a medical imaging apparatus and associated digital imaging and communications in medicine (DICOM) data, wherein the viewer application interacts with the plurality of medical image files, the computer program product comprising a non-transient computer readable storage medium having program code embodied therewith, the program code executable by a processor to:
identify, by a priority engine of the viewer application, a set of imaging studies of the plurality of imaging studies, wherein each of the set of imaging studies includes at least one medical image file requiring prefetching for a caching operation;
determine, by the priority engine, a priority value for each imaging study of the set of imaging studies based on a viewer application context associated with a respective imaging study;
store each imaging study in a study queue, a series queue, or an image queue within a memory of the PACS based on the corresponding priority value, wherein each imaging study is stored with:
a corresponding study index, a series index, and an image index,
a caching operation associated with the at least one medical image file of the imaging study requiring prefetching, and
a cache state of the plurality of medical image files of the imaging study;
query, by the priority engine, a priority cache for a next imaging study with at least one medical image file requiring prefetching;
receive, by the priority engine, a next study index corresponding to the imaging study with a highest priority value, an associated caching operation, and the cache state of the imaging study, wherein the next study index is the corresponding study index, the series index, and the image index;
identify, by the priority engine, the at least one medical image file of the imaging study with the highest priority value requiring prefetching;
prefetch, by the priority engine, the at least one medical image file of the imaging study with the highest priority value;
store, in an application cache within the memory of the PACS, the at least one prefetched medical image file of the imaging study with the highest priority value, wherein the application cache corresponds to the associated caching operation and the cache state of the imaging study with the highest priority value;
fetch at least one medical image file from the application cache when the associated caching operation is to be performed on the at least one medical image file; and
execute the associated caching operation on the medical image file.

9. The computer program product of claim 8, wherein the program code to identify the set of imaging studies of the plurality of imaging studies comprises program code to:
identify a first set of imaging studies within the set of imaging studies, the first set of imaging studies including at least one medical image file requiring prefetching for a first caching operation indicated by one or more user queries;
identify a second set of imaging studies within the set of imaging studies, the first set of imaging studies including at least one medical image file requiring prefetching for a second caching operation indicated by one or more user inputs selecting at least one imaging study;
identify a third set of imaging studies within the set of imaging studies, the third set of imaging studies including at least one medical image file requiring prefetching for a third caching operation indicated by a user subscription;
aggregate the first set of imaging studies, the second set of imaging studies, and the third set of imaging studies into the set of imaging studies of the plurality of imaging studies.

10. The computer program product of claim 8, wherein the priority value for each imaging study includes at least one of an image level priority value, a series level priority value, or a study level priority value, and
wherein the image level priority value is higher than the series level priority value, and wherein the series level priority value is higher than the study level priority value.

11. The computer program product of claim 8, wherein the viewer application context associated with the respective imaging study includes the associated caching operation, the cache state of the plurality of medical image files of the imaging study, and data indicating whether there is a user input or a user query associated with the imaging study.

12. The computer program product of claim 8, wherein the cache state of the image study comprises:
data indicating whether there are active operations for medical image files associated with the image study,
data indicating whether the image study and the associated medical image files are present in the application cache, or
data indicating eligibility of the image study and the associated medical image files for each caching operation.

13. The computer program product of claim 8, wherein the application cache comprises at least one of a decode image cache, an image file cache, or an image cache.
